# EUROPEAN PATENT SPECIFICATION

(11) **EP 4 426 242 B1**
(45) Date of publication and mention of the grant of the patent: **03.09.2025**
(21) Application number: 22803048.2
(22) Date of filing: 04.11.2022
(51) Int. Cl.: A61F 5/44

(54) **A CONNECTOR FOR CONNECTING TO AN OSTOMY APPLIANCE**
VERBINDER ZUR VERBINDUNG MIT EINER STOMAVORRICHTUNG
ÉLÉMENT DE LIAISON POUR LA LIAISON À UN ACCESSOIRE POUR STOMIE

(30) Priority: 05.11.2021 GB 202115928; 22.02.2022 GB 202202405
(43) Date of publication of application: 11.09.2024
(73) Proprietor: Salts Healthcare Limited, West Midlands B7 4AA (GB)
(72) Inventor: HOWARD, Lee, Birmingham, West Midlands B7 4AA (GB); SMITH, Lee, Birmingham, West Midlands B7 4AA (GB); JASICKA, Ewa, Birmingham, West Midlands B7 4AA (GB)
(74) Representative: Forresters IP LLP
(86) International application number: PCT/GB2022/052789
(87) International publication number: WO 2023/079298

(56) References cited:
- WO-A1-2017/127074
- US-A1- 2003 018 322
- US-A1- 2004 176 731

## Description

### Introduction

Embodiments of the present invention relate to a connector for connecting to an ostomy appliance. Ostomy appliances are well known medicals devices for users that have a stoma. Generally, the appliance has an opening for the stoma to be received in and an adhesive member that contacts the body and holds the appliance in position. Waste exiting the stoma is collected in a collecting volume, which is formed from two flexible walls connected together. Often the appliance includes an outlet valve, so that a user can empty waste that is collected in the collecting volume without having to remove and dispose of the entire appliance.

At times, a user may want to extend the time between emptying their appliance, for example, when they are sleeping. Typically, a larger capacity bag is used to contain waste while the user is sleeping. In this scenario, the outlet valve is connected to the larger capacity bag by a tube, so that the bag fills with waste.

WO2017/127074 discloses a stabilised catheter tube for insertion into a body of a patient. Embodiments of the present invention seek to alleviate one or more problems associated with the prior art.

According to a first aspect of the invention, we provide a connector for connecting to an ostomy appliance according to claim 1.

According to a second aspect of the invention, we provide a method of manufacturing a such connector for connecting to an ostomy appliance, according to claim 10.

Further preferred features relating to the aspects of the invention are provided in the appended claims.

### Detailed description

In order that the present disclosure may be more readily understood, preferable embodiments thereof will now be described, by way of example only, with reference to the accompanying drawings, in which:
FIGURE 1 is an illustrative view of an ostomy collection system.
FIGURE 2 is a perspective view of a connector;
FIGURE 3 is a partial cut-away view of the connector;
FIGURE 4A and 4B are illustrative cross-sectional views of a connector in use;
FIGRUE 5 is a side view of a connector;
FIGURE 6 is a cross-sectional view of a connector in accordance with another embodiment;
FIGURE 7A, 7B, 7C and 7D are cross-sectional views of a connector in accordance with another embodiment;
FIGURE 8 is a cross-sectional view of a connector in accordance with another embodiment;
FIGURE 9 is a side cross-sectional view of a connector in accordance with an embodiment; and
FIGURE 10 is a block diagram showing steps of a method of manufacture.

An ostomy device 1 (or ostomy appliance) is configured to attach around a stoma of a user (in the abdominal region), so that waste can be collected as it exits the stoma. The ostomy device includes an adhesive wafer, a collecting volume, and an outlet 1a. The adhesive wafer includes an opening through which the stoma passes and an adhesive which is suitable for adhering the wafer to the skin of the user. The collecting volume is formed of two outer walls attached about their periphery to define a volume within. The stoma opening connects to the collecting volume, so that when the device is attached to the user, waste exiting the stoma in the stoma opening is collected in the collecting volume.

The collecting volume is connected to the outlet 1a, which is selectively openable / closeable to drain waste from the collecting volume (into a toilet or other waste receptacle, for example). The outlet 1a may be formed in a "tail" portion of the device which is narrowed compared to the main collecting volume. There are different outlet designs that are designed to accommodate different types / consistencies of waste exiting the ostomy device 1.

Embodiments of the present invention are particularly suited for use with a waste with a high proportion of liquid (e.g. urostomy or ileostomy). In this situation, a valve or tap device is used as an outlet, which allows the waste to be emptied from the ostomy device as desired.

The outlet 1a includes a mechanism that allows further parts of a collection system to be attached. The collection system includes a secondary collection volume 2 that is connected to the ostomy device via a connector 10, so that waste may flow into the ostomy device 1 and through the connector 10 to the secondary collection volume 2.

The connector 10 includes a conduit 12 and a structural member 14. The conduit 12 has an entrance and an exit connected by an internal passage 16 (also called passage 16), through which waste is permitted to flow.

The conduit 12 is formed by a wall having an internal surface 12a and external surface 12b. The internal surface 12a defines the internal passage 16 (in other words, defines a perimeter of the passage 16). The external surface 12b forms the outside of the conduit 12. In some embodiments, the conduit 12 has a substantially constant diameter along its length (i.e. the diameter across the passage 16 is substantially constant). In some embodiments, the wall of the conduit 12 is a constant thickness, so the overall / external diameter of the conduit 12 is also substantially constant.

The internal diameter of the conduit 12 (i.e. the size of the passage) depends on the type of ostomy the connector 10 is designed to be used with. For example, both urostomy and ileostomy typically output a high proportion of liquid waste and, thus, a connector 10 as described here could be appropriate for transporting the waste. However, a urostomy may output waste at a slower rate whereas an ileostomy may output at a much faster rate. Thus, the connector 10 may be altered in size to provide an adequately sized passage 16 for differing output rates.

In some embodiments, the internal diameter of the conduit 12 is between around 6mm and 16mm. The internal diameter of the conduit 12 for use in urostomy may be at the low end of the range (i.e. between around 7 and 10mm and preferably around 8mm). Further, the diameter of the conduit 12 for use in ileostomy may be at the high end of the range (i.e. between around 13 to 16mm and preferably around 10mm)

In some examples (such as those illustrated in the figures), the conduit 10 is a flexible tube. The conduit 10 may be made of any suitable material that provides the necessary attributes, for example, a thermoplastic elastomer (TPE) or a thermoplastic polyurethane (TPU) may provide the necessary flexibility. A preferred material may be one that provides a rubber-like polymer.

In some embodiments, the conduit 12 forms a generally cylindrical shape. The flexibility of the conduit 12 allows it to change shape depending on the forces being put on the conduit 12 - however, it should be appreciated that the conduit 12 will return to its generally cylindrical shape once it is no longer being manipulated. For example, the conduit 12 can be bent, crushed and / or twisted, etc. and return to its initial shape once the forces causing that change in shape are removed (and preferably without damage).

A structural member 14 extends along the passage 16. The structural member 16 is present to prevent (or at least inhibit of) the passage 16 from being occluded. This may occur when the connector 10 is under pressure / being compressed, being twisted / bent, or otherwise manipulated in a way that causes the passage 16 to become blocked and / or causes waste to be inhibited from flowing through the passage 16.

In some embodiments, the structural member 14 extends along around half of a length of the passage 16. The structural member 14 may be deliberately inserted into the area / region of the passage 16 that is likely to be more susceptible to blockages. For example, the conduit 12 that is located on a generally horizontal surface (i.e. a mattress of a bed) may be more prone to blockage due to the lack of gravitational pull. The structural member 14 may be strategically positioned within the passage 16 to address this potential problem. Alternatively, the structural member 14 may extend along a majority of the length of the passage 16.

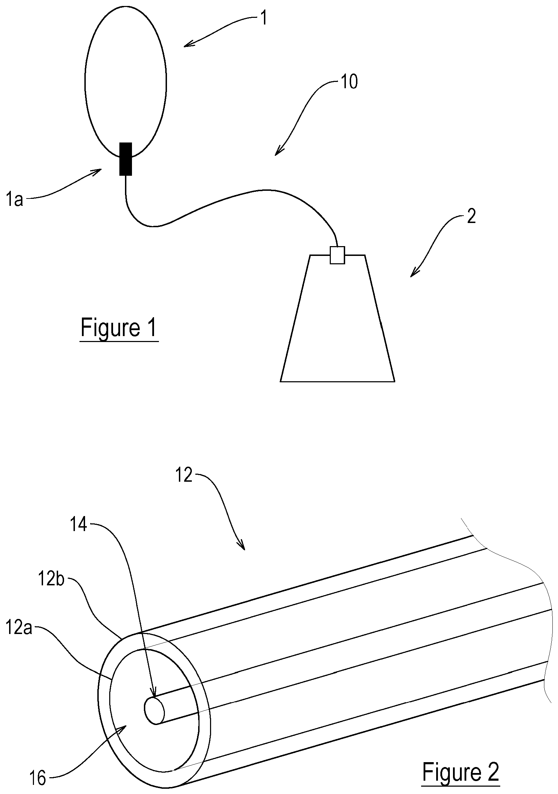

As illustrated in figures 2 to 5, the structural member 14 is a filament. In other words, the structural member 14 is a threadlike member or strand that extends along the passage 16. In the present example, the structural member 14 is formed substantially solidly, so that it resists being compressed (i.e. the structural member 14 does not provide any additional channel or flow path within the passage 16). In other words, the filament is less deformable than the conduit 12.

In some embodiments, the filament is less flexible than the conduit 12. This may also be beneficial because movement of the filament within the conduit 12 (as a result of the difference in flexibility), in use, may act to break up or disturb regions of more viscous waste (i.e. that otherwise may get stuck in the conduit 12 and slow down the passage of waste through the connector 10).

The filament structural member 14 is thin relative to the size of the passage (and preferably is a constant diameter along its entire length), so that it fits within the passage 16 easily. In some embodiments, the diameter of the filament may be between 1 to 2mm and preferably around 1.75mm to 2mm.

Thus, in a urostomy connector 10 as discussed above (e.g. a preferred diameters of around 8mm), the ratio of the diameter of the filament to the diameter of the passage 16 is around 2:8 to 7:32. In other words, the filament is under around 25% of the diameter of the passage 16. In embodiments, the diameter of the filament structural member 14 is not altered for use in an ileostomy connector 10 (e.g. having a diameter of around 10mm), so the ratio of the diameter of the filament to the diameter of the passage 16 may be about 2:10 to 7:40. In other words the filament is under around 20% of the diameter of the passage 16 - the filament may be smaller relative to the passage 16 in this case because the waste being handled may be at a higher rate or have a higher proportion of mucus / more solid waste.

In embodiments, the structural member 14 is made of a flexible material, so that it may flex / bend within the passage 16. For example, the filament may be made from the same material as the conduit 12 (e.g. a TPE or TPU or other rubber-like polymer). A possible suitable TPE is Styreneethylene-butylene-styrene (SEBS). Alternatively, a polylactic acid (PLA) may be used. However, a advantageous material for the filament may be polypropylene as it is less flexible than the conduit 12 around it and, as such, acts to prevent the conduit 12 from kinking.

Alternative flexible materials are available and preferably the material is PVC free and / or does not have a high coefficient of friction (e.g. is Silicon free). A low coefficient of friction is advantageous because it reduces the adherence between the structural member 14 and waste travelling through the passage 16.

Further, the filament is not absorbent, so waste is able to flow freely through the conduit 12 (around / past the filament) to the secondary collecting volume 2.

In some embodiments, the structural member 14 is not connected to the conduit 12 (or more specifically, is not attached / anchored to the internal surface 12a of the conduit) at any location. In other words, the structural member 14 is not held in a single axial position with respect to the conduit 12.

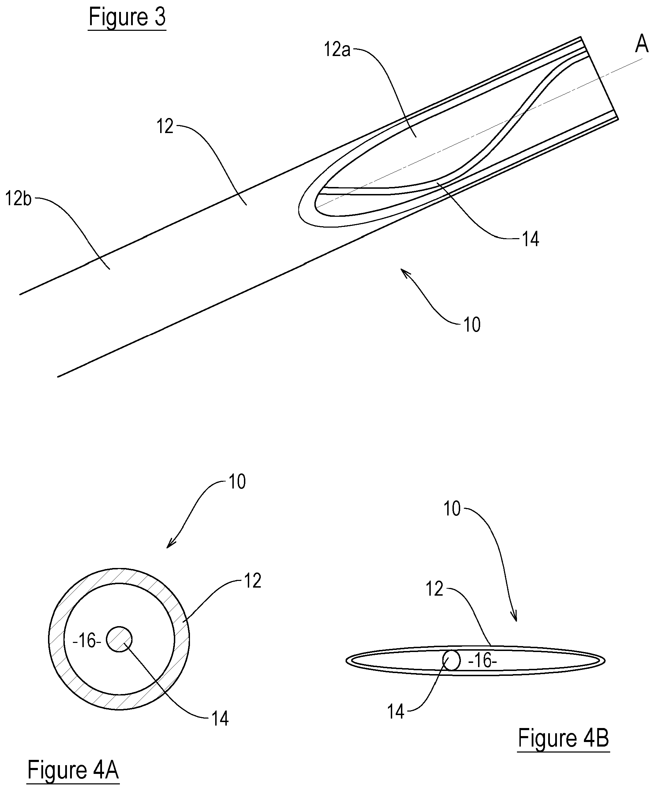

In some embodiments, the structural member 14 can move freely from side to side within the passage 16 (i.e. the structural member 14 can move generally transversely to the direction of the flow of waste through the passage 16). In other words, the conduit 12 has a longitudinal axis (e.g. axis A illustrated on figure 3) along the length of the connector 10 and the structural member 14 can move generally transversely to the axis. Furthermore, the structural member 14 may be flexible (and as such the structural member 14 may be manipulated to a more curved condition). This is illustrated in figure 3, for example, where the structural member 14 is shown to form a general sine wave shape that contacts the internal surface 12a on both "sides" of the passage 16.

As mentioned above, the structural member 14 may be unattached to the conduit 12. However, it should be appreciated that even if the structural member 14 were attached to the conduit 12 at two spaced positions along the length of the conduit, this would still allow the structural member 14 to move from side to side to some extent. The more anchor positions the structural member 14 had, the more restricted the radial movement of the structural member 14 would be. In some embodiments, one or more stake welds may be used to prevent the structural member 14 migrating along the conduit 12.

Thus, the structural member 14 may be free to move with respect to the conduit 12 (and relative to the axis A). This movement may be advantageous to maintaining waste transport along the conduit 12. For example, waste may include portions that have higher viscosity and do not flow easily along the conduit 12. The movement of the structural member 14 (i.e. movement of the filament from side to side of the passage 16) can be used in this situation to help break down regions of higher viscosity waste and aid the transport process / ensure the waste continues to flow away from the ostomy device 1. Additionally, the user is able to manipulate the conduit 12 to encourage the structural member 14 within to move from side to side and, thus, break down areas of waste with higher viscosity.

The structural member 14 includes a spanning formation 30 that extends radially across an increased width of the passage 16. In other words, the structural member 14 spans a wider diameter across the conduit 12 in one or more areas of the passage 16 than it does in the remainder of the passage 16. The spanning formation 30 is formed to provide resistance against axial movement of the structure member 14 along the passage 16 (i.e. in the direction of A from figure 3). The spanning formation 30 provides a transversely extending / projecting part(s) towards the opposing sides of the passage.

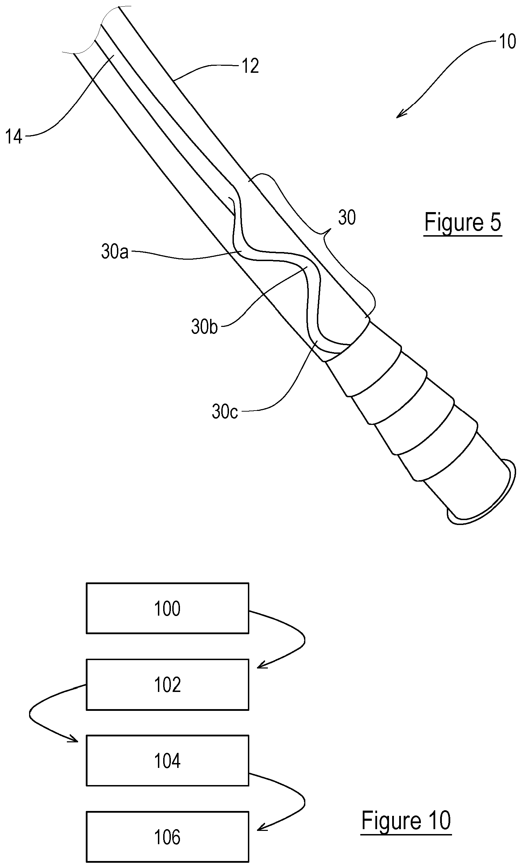

According to the invention (see, for example, figure 5), the spanning formation 30 is formed from a length of shaped filament. In other words, the elongate threadlike filament that extends along the passage 16 also includes a portion which is shaped to span a wider section of the passage 16. Thus, also the spanning formation 30 extends axially along the passage 16 over a short distance (e.g. around 3 to 5 cm).

In the illustrated example, the shaped filament is formed into a wave-like formation or a "zig-zag" formation. In other words, the shaping of the filament includes a plurality of "bends" formed in it to provide a portion that spans further widthways across the passage than the filament itself. A preferred example may include at least four bends.

It should be appreciated that the filament width (around 1.75mm, as discussed above) may not change in the spanning formation 30. However, the maximum width within the passage 16 covered by the spanning formation 30 (i.e. the "amplitude" of the wave, the distance between a maxima 30a, 30c on one side of the spanning formation 30 and the minima 30b on the other side of the spanning formation 30) is much wider than the filament itself.

In the present example, the spanning formation 30 (i.e. the shaped filament) is substantially centred about an axis along the filament. In other words, the spanning formation 30 extends generally radially outwardly from the filament - and in the illustrated design, the spanning formation 30 extends equally either side of the filament but this is not necessarily the case. In some embodiments, the spanning formation 30 is generally formed in a single plane. However, if desired, the formation 30 could be formed to extend in more than one plane.

In some embodiments, the spanning formation 30 extends across at least 80% of the diameter of the passage. However, it should be appreciated that the spanning formation 30 could extend across the entire passage 16 and contact the internal surface 16a of the passage. The spanning formation 30 may be wider than the diameter of the passage and, as such, the spanning formation 30 may slightly deform the natural shape of the conduit (i.e. the spanning formation extend up to around 105% of the undistorted diameter of the passage).

In some embodiments, the structural member 14 includes two such spanning formations 30 (in other words, there is a first and a second spanning formation 30 included in the connector 10 and they are spaced apart along the structural member 14). The first spanning formation 30 is positioned at one end of the structural member 14 (and possibly at an end of the conduit 12 also). The second spanning formation 30 may be located at a second / opposing end of the structural member 14 (and possibly at the opposing end of the conduit 12 also - if the structural member 14 extends substantially along the entire passage 16 length).

It should be appreciated that more spanning formations 30 could be provided if desired. In such a configuration the spanning formations 30 could be at either end of the conduit 12 and at one or more locations along the conduit 12.

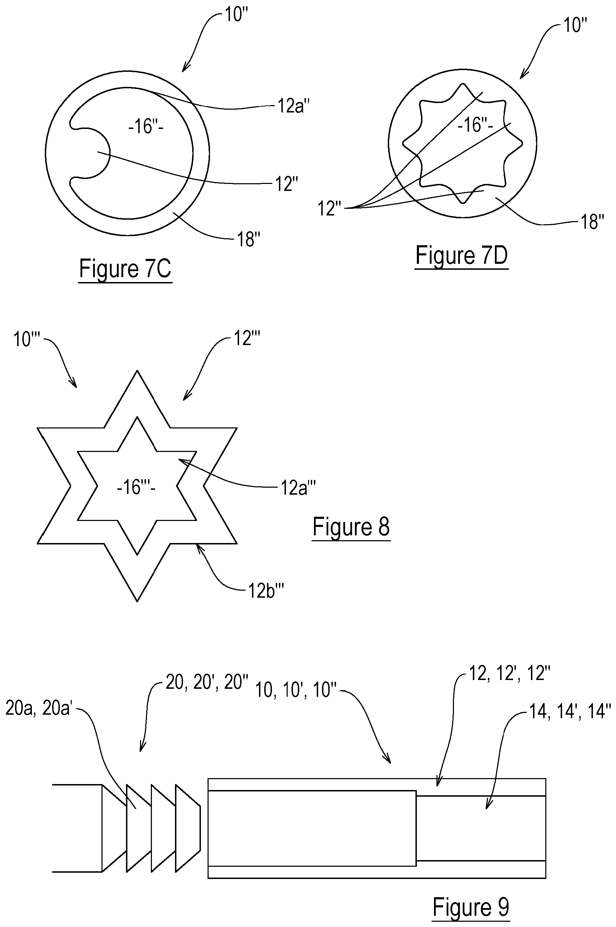

In some embodiments, at least one end of the conduit 12 is adapted to receive an adaptor device 20. The adaptor device 20 is configured to engage (via a liquid tight connection) with one of the outlet 1a of the ostomy device 1 and / or the inlet of the secondary collection volume 2. In the illustrated example in figure 8, the adaptor device 20 is inserted into the entrance and / or exit of the conduit 12. In this example, the adaptor device 20 includes a spigot type fitting 20a that is pushed into the passage 16 and, as such, the adaptor device 20 is held securely to the conduit 12. This ensures that the connector 10 provides a sealed transport path between the ostomy device 1 and the secondary collection volume 2.

In some embodiments, the adaption to the conduit 12 in order to receive the adaptor device 20 involves removing any parts of the structural member 14 from the "end" of the conduit 12 (i.e. an appropriate distance within the passage 16 adjacent to the entrance / exit). Thus, the conduit 12 has a generally unobstructed bore at one or both ends to allow the adaptor device 20 to be inserted without interruption from any parts present further down the passage 16. In other words, the length of the structural member 14 is shorter than the length of the conduit 12. Thus, the adaptor device 20 extends into the entrance and / or exit without being interrupted by the structural member 14.

It should be appreciated that not all adaptor devices will provide a male connection which is received by the conduit 12. If the adaptor device has a female connection that fits around the conduit 12, then it will not be necessary to adapt the ends of the conduit 12 to accommodate the adaptor device.

In some embodiments, the conduit 12 and the structural member 14 are formed separately (e.g. in two different extrusion lines). Subsequently, the structural member 14 is inserted into the conduit 12 to form the connector 10. In some embodiments, the conduit 12 and the conduit 14 are formed simultaneously in a co-extrusion machine.

An illustrative cross-sectional view of the connector 10 being used is shown in figures 4A and 4B. The figure 4A shows the conduit 12 in its natural round or annular state. The structural member 14 is shown in the centre of the conduit 12 for illustration only - the structural member 14 is able to move from side to side within the passage 16. Waste is free to travel allow the passage 16 around the structural member 14.

Figure 4B shows the conduit 12 when it is being compressed - this could arise because the connector 10 is being bent sharply / kinked and /or because it is being twisted / experiencing a torsional force and / or because it is under a compressing force (e.g. being stepped on). As can be seen in the figure, the conduit 12 no longer has its natural round / annular shape - it is forced into a shape resembling an oval or lanceolate. The structural member 14 within the passage 16 resists deformation and provides a reinforcing function that prevents the conduit 12 from compressing completely (i.e. prevents the internal surface 12a from either "side" of the conduit 12 from coming into contact). Thus, the flow path through the passage 16 is prevented (or at least inhibited) from closing / becoming occluded.

A method by which the embodiment illustrated in figures 2 to 5 is manufactured will now be described. Once the structural member 14 is located within the conduit 12 (step 100 in figure 10), further steps may be followed as described below.

A shaping tool is used at step 102 to shape a portion of the conduit 12 and structural member 14 simultaneously. In other words, the conduit 12 and the structural member 14 are deformed from an original or first shape by the shaping tool. Thus, both the conduit 12 and the structural member 14 form a second shape conforming to a profile of the shaping tool at the same time in the same step.

At step 104, the shaping tool is removed from the conduit 12 and the structural member 14. The structural member 14 maintains the second shape (i.e. the one formed in step 102 above). However, the conduit 12 returns to its original or at least approaches its original shape (i.e. before the shaping tool is used).

In embodiments, using the shaping tool (step 102) includes the application of pressure and / or heat onto the portion of the conduit 12 and the structural member 14 being shaped. Pressure is used to impress the shape of the tool onto the conduit 12 / structural member 14. Heat is used to raise the temperature of the conduit 12 and the structural member 14 during shaping. Importantly, a permanent deformation temperature of the structural member 14 is lower than the permanent deformation temperature of the conduit 12. In other words, the temperature above which the structural member 14 is moulded / the shape is permanently changed is lower than for permanently shaping the conduit 12.

This means when the shaping tool heats the conduit 12 and the structural member 14, it does so to a temperature which is above the permanent deformation temperature of the structural member 14 and below the permanent deformation temperature of the conduit 12. When the shaping tool is removed, only the structural member 14 is permanently shaped. In other words, the structural member 14 maintains the moulded shape from the shaping tool whereas the conduit 12 returns to its original (substantially tubular) configuration.

When using such a method, the materials of the conduit 12 and the structural member 14 have to carefully considered to ensure that the desired permanent shaping of the structural member 14 is formed while the conduit 12 still returns to its original shape after the shaping. Thus, while the conduit 12 may remain made of a TPE or TPU, the structural member 14 may be made of a material having a lower deformation temperature such as Ethylene-vinyl acetate (EVA) or PLA.

In some embodiments, the method includes cutting the conduit 12 and structural member 14. When small quantities of connectors 10 are made, the conduit 12 and the structural member 14 (the filament) may be cut to size and the structural member 14 is inserted in the conduit 12. However, in larger quantities, long lengths of filament may be inserted in the conduit 12 and kept in a prepared state ready for further processing (e.g. in a roll ready for use in a manufacturing machine(s)). Thus, the conduit 12 and filament may be found together in one continuous length and cut with a single action.

Furthermore, in some embodiments, cutting happens after using the shaping tool (at step 104). In other words, the shaping tool may be used to shape the filament along a longer length than needed for a single spanning formation 30 and the extended shaped filament is cut to provide the ends of two connectors 10 (each having a spanning formation 30 adjacent an end of the conduit 12). Thus, a double length spanning formation 30 is formed in one step and cut to provide two separate spanning formations 30.

Lastly, the method includes the step of inserting an adaptor device 20 in an end of the conduit 12 (step 106). As discussed above, the adaptor device 20 provides a connector for connecting directly to the outlet 1a of the ostomy appliance 1 or the secondary collection volume 2.

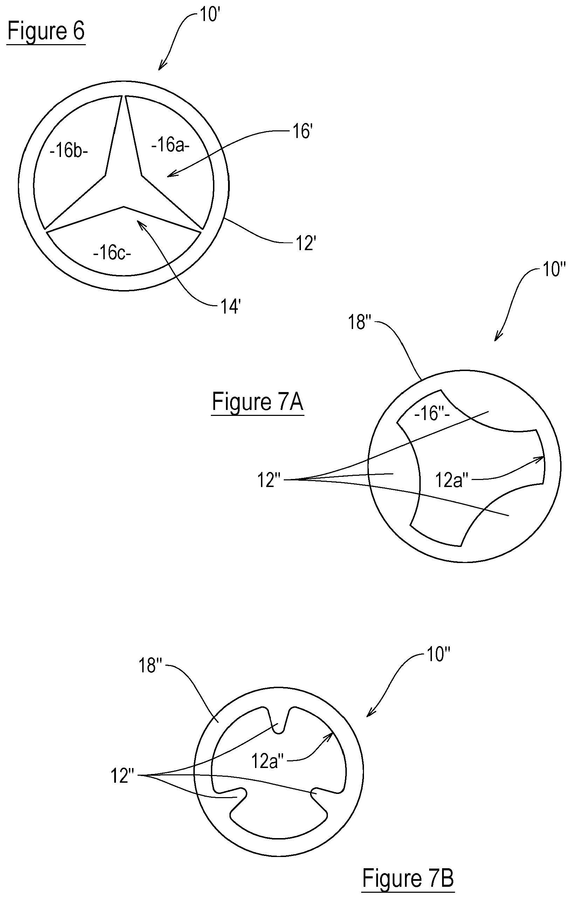

An alternative structural member is illustrated in figure 6. Where features are specifically described here and are analogous to features that have already been discussed, the reference numbers are the same with the addition of a prime symbol (e.g. 10 becomes 10'). The example illustrated in figure 6 only provides a cross-sectional view of the connector 10'. However, it should be appreciated that many of the features already described but not explicitly illustrated in figure 6 are still applicable, such as the materials used and the adaptions provided for an adaptor device 20'.

In some embodiments, the structural member 14' is formed to split the passage 16' into two or more channels. In other words, a specific shape of structural member 14' is chosen, so that it can be positioned in the passage 16'. The passage 16' is split by the structural member 14' to provide multiple lumens.

In some embodiments, the structural member 14' extends radially across the passage 16'. For example, the structural member 14' contacts an interior surface 12a' of the conduit 12' in at least two locations.

In the example illustrated in figure 6, the structural member 14' is formed with a cross-sectional shape of a three-pointed star. In cross section (i.e. across the conduit 12'), the points of the star extend outwards to contact the internal surface 12a' of the conduit 12' wall. In other words, the structural member 14' splits the passage 16' into three channels 16a, 16b, 16c. It will be appreciated that other shapes of structural member 14' could be used and achieve a similar outcome of multiple channels provided within the passage 16'. For example, a star shape having more than three points could be used to split the passage 16' into more than three channels. Furthermore, a structural member that engages the internal surface of the conduit 12' at only two locations could be used to split the passage 16' in two.

It should be appreciated that since the structural member 14' is flexible the surfaces contacting the internal surface 12a' of the conduit 12' does not provide a seal as such, so each channel formed is not necessarily sealed from the other channels. When the conduit 12' is twisted / bent / compressed / etc. the structural member 14' functions to strengthen the conduit 12' and resists deformation. Further, the portions of the structural member 14' that extend outwards to the internal surface of the conduit 12' (e.g. the points of the star shape) may inhibit major deformation and maintain one or more of the channels 16a, 16b, 16c within the passage 16' open for waste to flow.

An alternative embodiment of the disclosure is illustrated in figures 7A. Where features are specifically described here and are analogous to features that have already been discussed, the reference numbers are the same with the addition of a double prime symbol (e.g. 10 becomes 10"). New features also have a double prime but the references have not been used before. The example illustrated in figure 7 only provides a cross-sectional view of the connector 10". However, it should be appreciated that many of the features already described but not explicitly illustrated in figure 7 are still applicable, such as the materials used and the adaptions provided for an adaptor device.

The conduit 12" defines the internal passage 16" for the waste to flow along. In this example, the internal surface 12a" includes an inwardly projecting member 18".

In some embodiments, there are two or more inwardly projecting members 18" (three in the illustrated example), which are spaced around the internal surface 12a" of the conduit 12". The inwardly projecting members 18" may be equally spaced around the internal surface 12a" of the conduit 12".

In some embodiments, the inwardly projecting member(s) 18" extend longitudinally along the passage 16" (optionally, the inwardly projecting member(s) 18" extend along a majority of the total length of the conduit 12"). In other words, the inwardly projecting member 18" is present from the entrance to the exit of the conduit 12". Although it should be appreciated that they may not be directly adjacent the entrance and / or exit to accommodate an adaptor device 20" as already discussed above.

In the illustrated embodiment, the inwardly projecting members 18" are raised and smooth bumps that extend inwards from the internal surface 12a" of the conduit 12". However, it should be appreciated that this need not be the case and the shape of the projecting members 18" could be generally rectangular or another shape (discussed below). The important aspect is that the internal projecting member(s) 18" prevent or at least inhibit the passage 16" being occluded under compression / other manipulation as discussed in detail elsewhere in the description.

The examples illustrated in figure 7B to 7D are similar to that described above in that they include one or more projecting member(s) 18" extending radially inwards. The example in figure 7B also includes three inwardly projecting members 18". The inwardly projecting members 18" are generally triangular with a point extending furthest into the passage 16".

The example in figure 7C provides only a single inwardly projecting member 18". The member 18" is generally circular in cross-section with a portion attached to the internal surface 12a" of the conduit.

In the example in figure 7D, the internal surface 12a" of the conduit 12" is continuously undulate. This is formed from eight inwardly projecting members 18" but it should be appreciated that this could be adjusted depending on the size of the members 18" required and the size of the conduit 12".

An alternative conduit 12‴ is illustrated in figure 8. Where features are specifically described here and are analogous to features that have already been discussed, the reference numbers are the same with the addition of a triple prime symbol (e.g. 12 becomes 12‴). New features also have a triple prime but the references have not been used before. The example illustrated in figure 8 only provides a cross-sectional view of the connector 12‴. However, it should be appreciated that many of the features already described but not explicitly illustrated in figure 8 are still applicable, such as the materials used.

In this embodiment, the conduit 12'" includes an entrance and an exit connected by an internal passage 16'" through which waste is permitted to flow. The conduit 12'" has a polygonal cross-sectional shape. In some embodiments (as illustrated in figure 8), the conduit 12'" forms a sixpointed star shape in cross-section. However, it should be appreciated that a different shape could be used (for example, a star with a different number of points or another polygonal shape).

The conduit 12'" is formed by a wall having an internal surface 12a‴ and external surface 12b‴. The internal surface 12a‴ defines the internal passage 16‴. The external surface 12b‴ forms the outside of the conduit 12‴. In the present example, the wall is of substantially constant thickness along its length, so the diameter of the passage 16'" and conduit 12'" is substantially constant along the majority of the connector 10"'. In other words, both the internal shape of the wall and the external shape of the wall has the same cross-sectional shape.

In some embodiments, the conduit 12'" is formed of a suitable flexible material which permits an adaptor device to be inserted in the entrance and / or the exit. In this example, the conduit 12‴ is stretched / deformed to an annular shape to accept the adaptor device (not shown) (for example, a spigot fitting that is pushed into the deformed conduit 12‴). Additionally, the conduit 12'" seals against the adaptor device to inhibit egress of liquid between the conduit 12‴ and the adaptor device.

Embodiments of the disclosure described here provide a connector 10, 10', 10", 10‴ that resists occlusion in the event the conduit 12, 12', 12", 12‴ is compressed, twisted, bent, kinked, and/or other type of manipulation that could happen during use. Such occlusion could result waste becoming stuck in the passage 16, 16', 16", 16‴ for longer than necessary and / or backing up the passage 16, 16', 16", 16‴ and resulting in a leak at an earlier connection point (i.e. between the ostomy device and connector or in the ostomy device itself), which can be distressing to a user and / or cause a user to lose confidence in the system / connector provided.

Further, embodiments including a spanning formation 30 may be more advantageous since the spanning formation 30 provides a mechanism by which a filament within the conduit 12 resists unwanted migration along the conduit 12.

Although certain example embodiments of the invention have been described, the scope of the appended claims is not intended to be limited solely to these embodiments. The claims are to be construed literally, purposively, and/or to encompass equivalents.

## Claims

1. A connector (10) for connecting to an ostomy appliance including:
a conduit (12) having an entrance and an exit connected by an internal passage (16) through which waste from an ostomy appliance is permitted to flow, and
a structural member (14) including a filament, which extends along the passage, and a spanning formation (30) that extends radially across an increased width of the passage, and wherein the spanning formation (30) is formed from a length of shaped filament.

2. A connector (10) for connecting to an ostomy appliance according to claim 1 wherein the shaped filament is formed into a wave-like formation.

3. A connector (10) for connecting to an ostomy appliance according to claim 2 wherein the shaped filament has a plurality of bends formed into it and, optionally, has at least four external bends.

4. A connector (10) for connecting to an ostomy appliance according to claims 2 or 3 wherein the shaped filament is substantially centred about an axis along the filament.

5. A connector (10) for connecting to an ostomy appliance according to any one of the preceding claims wherein the spanning formation (30) is generally formed in a single plane.

6. A connector for connecting to an ostomy appliance according to any one of the preceding claims wherein the spanning formation extends axially along the passage.

7. A connector (10) for connecting to an ostomy appliance according to any one of the preceding claims wherein the structural member (14) includes a further, second, spanning formation (30), and optionally wherein the first and second spanning formations (30) are spaced from each other and, optionally, located at opposing ends of the conduit (12).

8. A connector for connecting to an ostomy appliance according to any one of the preceding claims wherein the spanning formation extends across at least 80% of the diameter of the passage and below 105% of the diameter of the passage.

9. A connector (10) for connecting to an ostomy appliance according to any one of the preceding claims wherein the structural member (14) has a lower permanent deformation temperature than that of the conduit (12).

10. A method of manufacturing a connector (10) according to claim 1, for connecting to an ostomy appliance, the method including the steps of:
using a shaping tool to shape a portion of the conduit (12) and structural member (14) simultaneously such that the conduit (12) and the structural member (14) forms a second shape conforming to a profile of the shaping tool; and
removing the shaping tool from the portion of the conduit (12) and structural member (14), and where the structural member (14) maintains the second shape.

11. A method according to claim 10 wherein the step of using a shaping tool on the portion of the conduit (12) and structural member (14) includes applying pressure to the portion and / or wherein the step of using a shaping tool on the portion of the conduit and structural member includes applying heat to the portion.

12. A method according to claim 11 wherein applying heat includes heating the portion to a temperature which is above a permanent deformation temperature of the structural member (14) and, preferably below a permanent deformation temperature of the conduit (12).

13. A method according to any one of claims 10 to 12 wherein the method includes inserting the structural member (14) in the passage of the conduit (12).

14. A method according to any one of the preceding claims wherein the method includes a step of cutting the conduit (12) and structural member (14) and preferably cutting both the conduit (12) and structural member (14) with a single action and optionally, wherein the cutting step occurs at the same time as or after using the shaping tool.

15. A method according to any one of claims 10 to 14 further including the step of inserting an adaptor in an end of the conduit (12), which provides a connector (10) for connecting directly to an outlet of an ostomy appliance or medical waste receptacle.

## Patentansprüche

1. Verbinder (10) zur Verbindung mit einer Stomavorrichtung, einschließend:
eine Leitung (12), die einen Eingang und einen Ausgang aufweist, die durch einen internen Durchtritt (16) verbunden sind, durch den Abfallstoffe von einer Stomavorrichtung strömen dürfen, und
ein Strukturelement (14), einschließend ein Filament, das sich entlang des Durchtritts erstreckt, und ein Spanngebilde (30), das sich radial über eine erhöhte Breite des Durchtritts erstreckt, und wobei das Spanngebilde (30) aus einer Länge eines geformten Filaments ausgebildet ist.

2. Verbinder (10) zur Verbindung mit einer Stomavorrichtung nach Anspruch 1, wobei das geformte Filament zu einem wellenartigen Gebilde ausgebildet ist.

3. Verbinder (10) zur Verbindung mit einer Stomavorrichtung nach Anspruch 2, wobei das geformte Filament eine Vielzahl von darin ausgebildeten Biegungen aufweist und optional mindestens vier externe Biegungen aufweist.

4. Verbinder (10) zur Verbindung mit einer Stomavorrichtung nach den Ansprüchen 2 oder 3, wobei das geformte Filament im Wesentlichen um eine Achse entlang des Filaments zentriert ist.

5. Verbinder (10) zur Verbindung mit einer Stomavorrichtung nach einem der vorhergehenden Ansprüche, wobei das Spanngebilde (30) im Allgemeinen in einer einzigen Ebene ausgebildet ist.

6. Verbinder zur Verbindung mit einer Stomavorrichtung nach einem der vorhergehenden Ansprüche, wobei sich das Spanngebilde axial entlang des Durchtritts erstreckt.

7. Verbinder (10) zur Verbindung mit einer Stomavorrichtung nach einem der vorhergehenden Ansprüche, wobei das Strukturelement (14) ein weiteres, zweites Spanngebilde (30) einschließt und optional wobei das erste und das zweite Spanngebilde (30) voneinander beabstandet sind und sich optional an entgegengesetzten Enden der Leitung (12) befinden.

8. Verbinder zur Verbindung mit einer Stomavorrichtung nach einem der vorhergehenden Ansprüche, wobei sich das Spanngebilde über mindestens 80 % des Durchmessers des Durchtritts und weniger als 105 % des Durchmessers des Durchtritts erstreckt.

9. Verbinder (10) zur Verbindung mit einer Stomavorrichtung nach einem der vorhergehenden Ansprüche, wobei das Strukturelement (14) eine niedrigere Dauerverformungstemperatur als die der Leitung (12) aufweist.

10. Verfahren zur Herstellung eines Verbinders (10) nach Anspruch 1 zur Verbindung mit einer Stomavorrichtung, wobei das Verfahren die Schritte einschließt:
Verwenden eines Formungswerkzeugs, um einen Abschnitt der Leitung (12) und des Strukturelements (14) gleichzeitig zu formen, so dass die Leitung (12) und das Strukturelement (14) eine zweite Form bilden, die einem Profil des Formungswerkzeugs entspricht; und
Entnehmen des Formungswerkzeugs aus dem Abschnitt der Leitung (12) und des Strukturelements (14), und wobei das Strukturelement (14) die zweite Form bewahrt.

11. Verfahren nach Anspruch 10, wobei der Schritt des Verwendens eines Formungswerkzeugs an dem Abschnitt der Leitung (12) und des Strukturelements (14) ein Anwenden von Druck auf den Abschnitt einschließt und/oder wobei der Schritt des Verwendens eines Formungswerkzeugs an dem Abschnitt der Leitung und des Strukturelements ein Anwenden von Wärme auf den Abschnitt einschließt.

12. Verfahren nach Anspruch 11, wobei das Anwenden von Wärme ein Erhitzen des Abschnitts auf eine Temperatur einschließt, die über einer Dauerverformungstemperatur des Strukturelements (14) und vorzugsweise unter einer Dauerverformungstemperatur der Leitung (12) liegt.

13. Verfahren nach einem der Ansprüche 10 bis 12, wobei das Verfahren ein Einführen des Strukturelements (14) in den Durchtritt der Leitung (12) einschließt.

14. Verfahren nach einem der vorhergehenden Ansprüche, wobei das Verfahren einen Schritt eines Schneidens der Leitung (12) und des Strukturelements (14) und vorzugsweise eines Schneidens sowohl der Leitung (12) als auch des Strukturelements (14) mit einer einzigen Aktion einschließt und optional wobei der Schneidschritt zur selben Zeit wie das oder nach dem Verwenden des Formungswerkzeugs erfolgt.

15. Verfahren nach einem der Ansprüche 10 bis 14, ferner einschließend den Schritt eines Einführens eines Adapters in ein Ende der Leitung (12), der einen Verbinder (10) zur direkten Verbindung mit einem Auslass einer Stomavorrichtung oder einem Behälter für medizinische Abfallstoffe bereitstellt.

## Revendications

1. Connecteur (10) destiné à être connecté à un appareillage de stomie comprenant :
un conduit (12) ayant une entrée et une sortie reliées par un passage interne (16) à travers lequel les déchets provenant d'un appareillage de stomie peuvent s'écouler, et
un élément structurel (14) comprenant un filament, qui s'étend le long du passage, et un recouvrement (30) qui s'étend radialement sur une plus grande largeur du passage, et dans lequel le recouvrement (30) est formé à partir d'un longueur du filament façonné.

2. Connecteur (10) destiné à être connecté à un appareillage de stomie selon la revendication 1, dans lequel le filament façonné est de forme ondulée .

3. Connecteur (10) destiné à être connecté à un appareillage de stomie selon la revendication 2, dans lequel le filament façonné a une pluralité de courbes formées dans celui-ci et, de manière optionnelle, comporte au moins quatre courbes externes.

4. Connecteur (10) destiné à être connecté à un appareillage de stomie selon les revendications 2 ou 3, dans lequel le filament façonné est sensiblement centré autour d'un axe le long du filament.

5. Connecteur (10) destiné à être connecté à un appareillage de stomie selon l'une quelconque des revendications précédentes, dans lequel le recouvrement (30) est généralement formé dans un seul plan.

6. Connecteur destiné à être connecté à un appareillage de stomie selon l'une quelconque des revendications précédentes, dans lequel le recouvrement s'étend axialement le long du passage.

7. Connecteur (10) destiné à être connecté à un appareillage de stomie selon l'une quelconque des revendications dans lequel l'élément structurel (14) inclut un autre, second, recouvrement (30), et de manière optionnelle dans lequel les premier et second recouvrements (30) sont éloignés l'un de l'autre et, de manière optionnelle, situés aux extrémités opposées du conduit (12).

8. Connecteur destiné à être connecté à un appareillage de stomie selon l'une quelconque des revendications précédentes, dans lequel le recouvrement s'étend sur au moins 80 % du diamètre du passage et sur moins de 105 % du diamètre du passage.

9. Connecteur (10) destiné à être connecté à un appareillage de stomie selon l'une quelconque des revendications précédentes, dans lequel la température de déformation permanente de l'élément structurel (14) est inférieure à celle du conduit (12).

10. Procédé de fabrication d'un connecteur (10) selon la revendication 1, destiné à être connecté à un appareillage de stomie, le procédé comprenant les étapes consistant à :
utiliser un outil de mise en forme afin de mettre en forme une partie du conduit (12) et de l'élément structurel (14) simultanément de sorte que le conduit (12) et l'élément structurel (14) prennent une seconde forme conforme à un profil de l'outil de mise en forme ; et
retirer l'outil de mise en forme de la partie du conduit (12) et de l'élément structurel (14), et où l'élément structurel (14) garde la seconde forme.

11. Procédé selon la revendication 10 dans lequel l'utilisation d'un outil de mise en forme sur la partie du conduit (12) et l'élément structurel (14) comprend l'application d'une pression sur la partie et/ou dans lequel l'étape d'utilisation d'un outil de mise en forme sur la partie du conduit et l'élément structurel comprend l'application de chaleur sur la partie.

12. Procédé selon la revendication 11, dans lequel l'application de chaleur comprend le réchauffement de la partie à une température supérieure à une température de déformation permanente de l'élément structurel (14) et, de préférence inférieure à une température de déformation permanente du conduit (12).

13. Procédé selon l'une quelconque des revendications 10 à 12, dans lequel le procédé comprend l'insertion de l'élément structurel (14) dans le passage du conduit (12).

14. Procédé selon l'une quelconque des revendications précédentes, dans lequel le procédé comprend une étape consistant à couper le conduit (12) et l'élément structurel (14) et, de préférence, à couper le conduit (12) et l'élément structurel (14) dans une même action et de manière optionnelle, dans lequel l'étape de coupe est réalisée conjointement à ou après l'utilisation de l'outil de mise en forme.

15. Procédé selon l'une quelconque des revendications 10 à 14, comprenant en outre l'étape d'insertion d'un adaptateur sur une extrémité du conduit (12), qui forme un connecteur (10) destiné à être connecté directement à une sortie d'un appareillage de stomie ou un réceptacle de déchets médicaux.
